Europäisches Patentamt

European Patent Office

Office européen des brevets

(11) Publication number: **0 133 321 B1**

(12) **EUROPEAN PATENT SPECIFICATION**

(45) Date of publication of patent specification: **16.09.92** (51) Int. Cl.⁵: **C12N 15/62**

(21) Application number: **84109016.0**

(22) Date of filing: **30.07.84**

(54) **DNA gene, process for production thereof and plasmid containing the same.**

(30) Priority: **01.08.83 JP 140748/83**

(43) Date of publication of application:
**20.02.85 Bulletin  85/08**

(45) Publication of the grant of the patent:
**16.09.92 Bulletin  92/38**

(84) Designated Contracting States:
**CH DE FR GB IT LI SE**

(56) References cited:
**EP-A- 0 038 182**

(73) Proprietor: **WAKUNAGA SEIYAKU KABUSHIKI KAISHA
2-14, Fushimi-Machi, 4-Chome, Chuo-Ku
Osaka-shi Osaka-fu(JP)**

(72) Inventor: **Miyaka, Tetsuo
706-1, Kamikoutachi Kouda-Cho
Takata-Gun Hiroshima-Ken(JP)**
Inventor: **Oka, Takanori
479, Ku, Koyo-Cho Asakita-Ku
Hiroshima-Shi Hiroshima-Ken(JP)**
Inventor: **Tamura, Gakuzo
17-12, Sanno 2-Chome
Ota-Ku Tokyo-To(JP)**
Inventor: **Yoda, Koji**

**22-15, Mejiro 5-Chome
Toshima-Ku Tokyo-To(JP)**
Inventor: **Kikuchi, Yasuhiro
Kurumi-So 5-27-3, Daita
Setagaya-Ku Tokyo-To(JP)**
Inventor: **Yamasaki, Makari
4-102, Fuchu Daini Danchi 2-1-10, Harumi-Cho
Fuchu-Shi Tokyo-To(JP)**

(74) Representative: **Kraus, Walter, Dr. et al
Patentanwälte Kraus, Weisert & Partner
Thomas-Wimmer-Ring 15
W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to utilization of a DNA gene participating in secretion of an exogenous or foreign gene product. More specifically, the present invention pertains to a DNA comprising a gene coding for a peptide for excreting the protein produced, as the expression of said DNA gene, within cells of a host microorganism through the membrane, which peptide will hereinafter be referred to as "signal peptide", which DNA portion is so processed that an exogenous gene can be linked directly to said DNA gene. It also relates to a process for producing the DNA gene and also to a plasmid containing the DNA gene portion.

Techniques for producing a large amount of a desired gene product by the use of recombinant DNA technology are now being established, as evidenced by the large number of related reports and published patent specifications. With the establishment of these techniques, genetic analyses have also been made, and the knowledge that a series of peptides comprising 15 to 30 amino acids participate in secretion of an expressed protein out of the cell of a host has also been gained from among such analyses.

The knowledge concerning secretion of the expressed protein out of cells is called "Signal Hypothesis" [J. Cell Biol., 67, 835 (1975)], and the experimental results in support of this hypothesis are being accumulated [Secretory Mechanisms, 9, Cambridge University Press, Cambridge (1979); Seikagaku (Journal of Biochemistry), 52, 141 (1980), etc.]. The outline of the signal peptide and its function are described in, for example, Japanese Laid-open Patent Publication No.69897/1983, and the signal peptide is also recognized as participating in the permeation of proteins through membranes.

At present, various inventions utilizing signal peptides are disclosed in Laid-open or Published Patent Publications (e.g., Japanese Laid-open Patent Publications Nos. 19092/1980, 45395/1980, 137896/1981, 145221/1981 and 154999/1981). According to any of the processes disclosed in these publications, the structural gene coding for the protein to be secreted by a host microorganism is cleaved at a suitable restriction endonuclease recognition site, and an exogenous gene is connected thereto through a linker for matching to the frame. As a result, the exogenous protein upon expression of such a fused gene has at its N-terminus side a superfluous peptide attached. Accordingly, there is a possibility that such a superfluous peptide might inhibit secretion or have a deleterious effect on the biological activity of exogenous protein.

EP-A-38 182 describes that a signal peptide with an exogenous excretable protein inherently has functioned in an E. coli to secrete the protein downstream to the signal peptide as a mature protein.

Thus, the techniques of the prior art utilizing signal peptides involve various problems and it would be desirable to solve these problems.

It is an object of the present invention to overcome the above described problems, and it is intended to accomplish this object by designing and utilizing a DNA gene which will make possible linking of a gene coding for an exogenous protein to the gene coding for a signal peptide immediately thereafter.

Accordingly, according to the present invention there is provided a DNA comprising a gene coding for a natural bacterial signal peptide, said gene differing from the corresponding natural gene by having, towards its downstream end, at least part of a restriction endonuclease recognition site artificially created from the natural gene by using the degeneracy of the genetic code so that the same amino acid sequence is encoded.

A process for producing a DNA as defined above comprises the steps of:
(a) providing a DNA containing a gene coding for a natural bacterial signal peptide; and
(b) modifying the base sequence of said gene towards its downstream end without changing the amino acid sequence corresponding to said base sequence, thereby creating a restriction endonuclease recognition site at least a part of which is located on said gene.

In the above, step (b) can be followed by
(c) cleaving the DNA with the restriction endonuclease recognized by said site, thereby obtaining a cleaved end of said restriction endonuclease;
(d) providing another DNA fragment having the same restriction endonuclease cleaved end on its upstream terminus, followed, if necessary, by a DNA portion useful to reconstitute the gene for the natural bacterial signal peptide, the latter being followed by an exogenous gene; and
(e) linking the DNA fragments from the above steps (c) and (d) to each other at the restriction endonuclease cleaved ends, thereby obtaining a DNA comprising a gene coding for a natural bacterial signal peptide and the exogenous gene directly linked to the downstream terminus of the signal peptide gene.

The plasmid according to the present invention comprises the controlling region derived from an alkaline phosphatase gene and a gene coding for the alkaline phosphatase signal peptide, said gene coding for the signal peptide being defined by having a restriction endonuclease recognition site artificially created such that the amino acid sequence coded for by the base sequence is not changed and that the corresponding cleavage site lies at the downstream terminus of said gene.

Thus, in order to afford extracellular excretion of proteins from exogenous genes by a signal peptide, the present invention has a specific feature in that a restriction endonuclease recognition/cleavage site is created for incorporation of an exogenous gene into the signal peptide gene, and the creation of this site is based on skillful utilization of the fact that there is degeneracy in codons comprising DNA base pairs.

When cleavage is effected at the created restriction endonuclease recognition/cleavage site by the restriction endonuclease concerned, and in the case where the cleavage site exists contiguous to the downstream terminus of the signal peptide gene DNA, it follows that an exogenous gene having at its upstream terminus a DNA fraction complementary to said restriction endonuclease cleaved end formed is prepared and is linked at the cleaved end to the signal peptide gene, whereby the exogenous gene will be linked directly to the downstream terminus of the signal peptide gene. In the case where the cleavage site of the signal peptide gene is located not at its downstream terminus but upstream to its downstream terminus and cleavage thus takes place at a place upstream to the downstream terminus of the signal peptide gene resulting in disintegration of the signal peptide gene, a DNA fragment comprises a DNA (p) for an exogenous gene and a DNA (q) linked to the upsteam terminus of the DNA (p) gene, which DNA (q) corresponds to the DNA portion of the DNA for the signal peptide gene downstream from the cleavage site, and the DNA fragment is then linked to the cleaved end of the signal peptide gene, whereby the signal peptide gene once disintegrated by the cleaving will be restored for both strands of DNA simultaneously with realization of a structure wherein the exogenous gene is linked directly to the downstream end of the signal peptide.

The DNA gene we have produced can thus circumvent the above problems when it is utilized as a vector incorporated in a suitable plasmid or phage and has the following advantages when incorporated into a host microorganism for production of a desired protein.

(a) Purification of the expression products formed is easy. In the prior art, the entire host cell was crushed at an appropriate stage after growth of the host microbe, and useful substances were purified by extraction from among various cell contents of the microbe used. Accordingly, an enormous amount of labor was necessary, and also purification was sometimes difficult depending on the substance being purified. By the use of a vector utilizing the DNA gene of the present invention, the protein produced is excreted out of the microorganism cells, and it is thus easy to identify and recover the desired substance secreted from the medium since the constituents of the growth medium for the microorganism are known.

(b) The substance produced can be protected from decomposition by peptidase. More specifically, because of the presence of much peptidase (protease) within the microbial cells, unnecessary proteins are subject to rapid hydrolysis, but immediate excretion of the desired useful protein out of microbial cells without undue retention therein will lead to protection of the desired protein from the above hydrolase.

(c) Any exogenous protein can be expressed. For example, according to the fused protein method of the prior art, such a typical method for obtaining the desired protein out of the fused protein by cleaving the fused protein at the site where the desired protein is fused with an undesired protein as treatment of the fused protein with cyanogen bromide which is specific to methionine or as tripsin digestion of the fused protein, trypsin being specific to lysine or arginine, is applicable only to proteins which do not contain methionine, lysine or arginine. On the other hand, when producing a protein according to the direct expression method [Nature 281, 544 (1979)], an initiation codon (methionine) is required to exist at the N-terminus of the gene, and some proteins produced are obtained with methionine being attached at the N-terminus (see Japanese Laid-open Patent Publication No. 68399/1981). Because it is technically difficult to remove by decomposition such methionine at the terminus by cyanogen bromide treatment, the protein produced having the methionine is consequently different in nature from the natural product. In contrast, when the exogenous gene is expressed in a host microbe with the use as a vector of the DNA gene according to the present invention, the proteins produced once as fused or hybrid protein with a signal peptide are cleaved specifically at the signal peptidase portion to provide a mature protein with a desired composition secreted out of the host microorganism cells.

In the drawings:

FIG. 1 is a diagram of the vicinity of the linked site between the gene encoding a natural bacterial signal peptide (1) and the structural gene (2), indicating the restriction endonuclease recognition site (3), the restriction cleavage site (broken line), and the signal peptide cleavage point (4);

FIG. 2 is a flow chart for the production of the plasmid pTA 529 of the present invention;

FIGS. 3A and 3B are facsimiles of autoradiograms obtained when colony hybridization was carried out;

FIG. 4 is a diagram indicating the result of agarose gel electrophoresis; and

FIG. 5 is a facsimile of an autoradiogram obtained when the base sequence was determined according to the Maxam-Gilbert method.

DNA gene

The DNA comprising a gene coding for a natural bacterial signal peptide according to the present invention (in the present specification, particularly in the description hereinafter, "DNA corresponding to the gene coding for a natural bacterial signal peptide" is referred to as "natural bacterial signal peptide gene DNA") has one restriction endonuclease recognition site artifically created in the DNA, as mentioned above. And, the restriction endonuclease recognition site contains at least one base pair of the DNA as at least a part thereof.

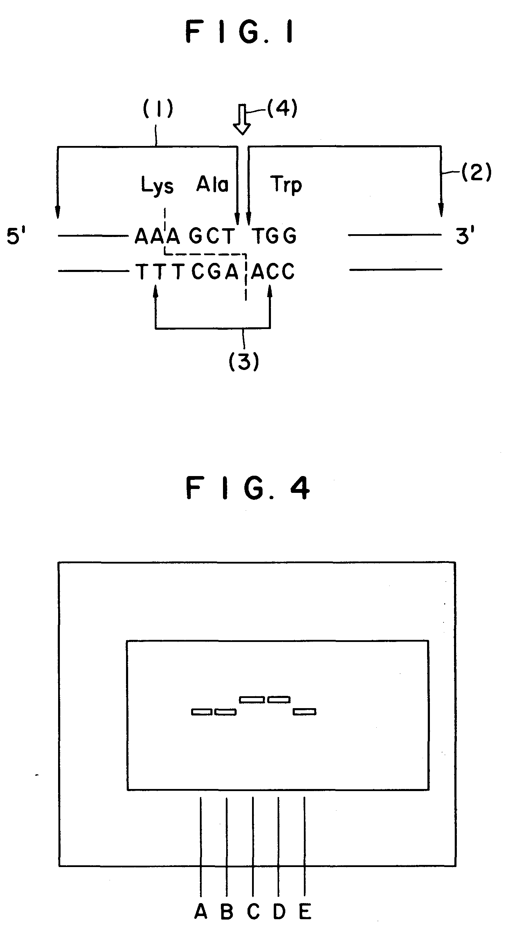

An example of the DNA gene according to the present invention thus defined is as shown in FIG. 1. This embodiment comprises the natural bacterial signal peptide gene DNA portion (1) and the DNA portion (2) linked thereto on its downstream side terminus. The "DNA gene containing the DNA portion corresponding to the gene coding for a natural bacterial signal peptide" as mentioned herein may comprise at least the DNA portion (1). In the present invention, the term "downstream" when it is concerned with DNA means the direction to the right or location at the right when the 5' → 3' chain ( ⊕ chain) is shown above and the 3' ← 5' chain ( ⊖ chain) is shown below. The term "upstream" will then be self-explanatory.

FIG. 1 shows a part of the DNA gene comprising a double-stranded DNA, in which A, G, C and T stand for adenine, guanine, cytosine and thymine, respectively, and Lys, Ala and Trp lysine, alanine and tryptophane, respectively. The region (1) of the double-stranded DNA corresponds to the natural bacterial signal peptide gene DNA portion, the region (3) to the recognition site of the restriction endonuclease Hind III, and the broken line to the Hind III cleavage site. The region (2) corresponds to the DNA portion linked to the natural bacterial signal peptide immediately thereafter on its downstream side.

According to a preferred embodiment of the present invention, the natural bacterial signal peptide gene DNA is one which has originated from an alkaline phosphatase. This DNA has a codon of GCC for the alanine at its downstream terminus.

The DNA gene shown in FIG. 1, therefore, corresponds to one obtained by modifying the codon GCC at the downstream terminus of the DNA portion (1) from alkaline phosphatase to GCT and further the subsequent base C to T. Since there is degeneracy in the codon for alanine, GCT after modification is also the codon for alanine, and therefore the DNA portion (1) in FIG. 1 is still a DNA corresponding to the gene coding for the signal peptide from alkaline phosphatase.

The natural bacterial signal peptide gene DNA from alkaline phosphatase has, just upstream to the codon for alanine at its downstream terminus, a codon AAA for lysine, which codon for alanine is accompanied by, just downstream from it, a codon CGG for arginine.

Accordingly, upon modification into GCT of the codon GCC for alanine at the downstream terminus of the natural bacterial signal peptide gene DNA from alkaline phosphatase and modification into T of the base C subsequent to the alanine, a recognition site (3) AAGCTT for the restriction endonuclease Hind III is created, namely from the base pair at the downstream terminus of the signal peptide gene DNA, 4 base pairs upstream thereto and one base pair downstream therefrom. Thus, there is created in the natural bacterial signal peptide gene DNA a restriction endonuclease recognition site containing at least the base pair at said terminus as at least a part of its constituting members.

In the embodiment of the invention shown in FIG. 1, the cleavage site within the recognition site (3) of Hind III is as shown by the broken line, and its position exists between the natural bacterial signal peptide gene DNA and the DNA portion which is to be linked thereto immediately thereafter on its downstream side (the region (2) already linked in FIG. 1) (the position of the cleavage site means that on the downstream side of the double-stranded DNA). It is most preferable in the present invention that the restriction endonuclease cleavage site exist at such a position. This is because of the following reason. The cleavage site is utilized to link an exogenous gene (DNA corresponding thereto) directly to the gene coding for the natural bacterial signal peptide (DNA corresponding thereto), while the fused or hybrid protein formed by expression of the hybrid gene is cleaved by the signal peptidase at a position between the natural bacterial signal peptide and the protein subsequent thereto. Therefore, if the restriction endonuclease cleavage site is thus coincident with the signal peptidase cleavage site, and in the case of the embodiment of the invention shown in FIG. 1, only supplementing AGCT to the 5'-side of the ⊕ chain of the exogenous gene (in this embodiment, beginning with the codon TGG for Trp), is required for making it possible to link the exogenous gene to the cohesive end of the natural bacterial signal peptide gene after digestion with Hind III. In this connection, if one is not reluctant to supplement bases to the 3'-side of the ⊖ chain of the exogenous gene, the restriction endonuclease cleavage site can exist on the upstream side, of course, and existence of such a cleavage site is also included within the scope of the present invention.

The "DNA corresponding to the gene coding for the natural bacterial signal peptide", which is the important component in the DNA gene of the present invention, may have a variety of base sequences depending on the natural bacterial signal peptide employed. Specific examples of natural bacterial signal peptide are that of $\beta$-lactamase [Proc. Natl. Acad. Sci. U.S.A., 75, 3737 (1978)], that of lipoprotein [ibid, 74, 1004 (1977)], and that of alkaline phosphatase [Eur. J. Biochem., 96, 49 (1979)]. Concerning signal peptides, reference may be made to "Tampakushitsu•Kakusan•Koso" (Protein, Nucleic Acid and Enzyme), extra edition ("Genetic Manipulation"), vol. 26, No.4, pages 386-394. In the present invention, these signal peptides originating from natural products as well as DNA's synthesized according to the base sequences thereof can be used (the former being preferred).

The natural bacterial signal peptide gene DNA preferably used in the present invention is one having the base sequence of alkaline phosphatase, particularly one which originates from alkaline phosphatase. This is because the advantages as described with reference to FIG. 1 can be obtained.

In one embodiment of the DNA gene of the present invention as shown in FIG. 1, which has been prepared, as described in detail below, by modification of the DNA from alkaline phosphatase, the DNA portion (2) from alkaline phosphatase is linked to the natural bacterial signal peptide gene DNA portion (1) immediately after its downstream terminus. Another embodiment of the DNA gene according to the present invention is one wherein the DNA portion (2) is the DNA portion corresponding to the gene coding for the exogenous protein. To abide by the purpose for introducing the restriction endonuclease cleavage site, the embodiment in which the DNA portion (2) originates from an exogenous gene may be said to be a specific example conforming to the purport of the invention.

An example of the DNA gene of the present invention encompassed within the category of the latter embodiment thereof comprises the natural bacterial signal peptide gene DNA portion, constituted by the moiety from a natural product and a moiety which is synthesized. More specifically, the moiety upstream to the restriction endonuclease cleavage site included is one from a natural product, while the moiety downstream from the cleavage site is one synthesized. The synthesized moiety downstream from the cleavage site in the example shown in FIG. 1 is the four base (AGCT) of the ⊕ chain, but a synthesized moiety is required also in the ⊖ chain, if the cleavage site exists upstream to that position shown.

Production of DNA gene

The process for production of the DNA gene encoding the signal peptide gene comprises the steps of:
(a) providing a DNA containing a gene coding for a natural bacterial signal peptide; and
(b) modifying the base sequence of said gene towards its downstream end without changing the amino acid sequence corresponding to said base sequence, thereby creating a restriction endonuclease recognition site at least a part of which is located on said gene.

The process for producing the DNA gene according to the present invention may alternatively be comprehended as a method of modifying a given DNA gene.

As described above, the DNA gene conforming to the purport of the present invention has a DNA portion originating from an exogenous gene present immediately after the downstream terminus of the natural bacterial signal peptide gene. Such a DNA gene can be produced by practicing the steps (c) to (e) set forth below for the DNA gene as prepared above:
(c) cleaving the DNA with the restriction endonuclease recognized by said site, thereby obtaining a cleaved end of said restriction endonuclease;
(d) providing another DNA fragment having the same restriction endonuclease cleaved end on its upstream terminus, followed, if necessary, by a DNA portion useful to reconstitute the gene for the natural bacterial signal peptide, the latter being followed by an exogenous gene; and
(e) linking the DNA fragments from the above steps (c) and (d) to each other at the restriction endonuclease cleaved ends, thereby obtaining a DNA comprising a gene coding for a natural bacterial signal peptide and the exogenous gene directly linked to the downstream terminus of the signal peptide gene.

The "DNA fragment of (d)" is not essential in that it is not required when the cleavage site is at the downstream terminus of the natural bacterial signal peptide DNA, but is required when the cleavage site is located upstream to the downstream terminus of said DNA and there is thus a DNA portion between the cleavage site and the downstream terminus of said DNA , which DNA portion is cleaved off when cleavage is performed and is thus required for restoring the once disintegrated natural bacterial signal peptide DNA when linking of the exogenous gene is performed.

For creation of a restriction endonuclease recognition site by modification of at least one base pair of the natural bacterial signal peptide gene DNA, and, if required, the gene DNA downstream from the natural bacterial signal peptide, any method suited for the purpose may be employed.

As the method for modification of base sequence, a method such as the point mutation method employing a synthetic fragment, the method employing a mutation inducer (X-ray, N-methyl-N'-nitro-N-nitrosoguanidine, etc.), the method in which mutation is caused by use of sulfite ions or nitrite ions, or the method through replacement with a synthesized gene may be considered. However, as a method which is simple and reliable in obtaining a desired DNA gene, it is preferable to use the point mutation method employing a synthetic fragment [Science, 29, 19, September (1980)].

The essential point in the modification of the base sequence is to create a restriction endonuclease recognition site without changing the amino acid sequence within a given signal peptide. The restriction endonuclease recognition site, which is within the gene coding for the signal peptide, should preferably be as close as possible to the cleavage point of the signal peptide by signal peptidase, more preferably its cleavage site being coincident with the cleavage point of the signal peptide, as described above. According to the most preferred embodiment, as shown in FIG. 1, the codon GCC at the downstream terminus of the base sequence (1) coding for the natural bacterial signal peptide of alkaline phosphatase is modified into GCT, whereby the amino acid sequence is not changed by this modification since GCC and GCT are both coding for alanine, and, further, the base C just downstream from the gene (1) for the signal peptide is modified into T to create the restriction endonuclease Hind III recognition site. In this case, the cleavage point (shown by the arrowhead (4)) and the restriction endonuclease cleavage site (broken line) coincide with each other. Accordingly, linking of a desired exogenous structural gene to the signal peptide immediately thereafter is rendered possible. In this case, the restriction endonuclease cleaved end is a cohesive end. By providing a desired exogenous gene with a cohesive end of AGCT at its upstream terminus, it is possible to obtain a fused protein in which the signal peptide and the exogenous protein are linked directly together to accomplish the above object, as described above.

Utilization of DNA gene

The DNA gene of the present invention can be utilized as a vector in such a form that a desired exogenous structural gene is linked to the DNA gene and that, when necessary, it is inserted into a plasmid or a phage at an appropriate position downstream to its promoter. By introducing an exogenous gene incorporated in the vector into a host microorganism such as E. coli, and culturing the host microorganism, the protein formed by expression of the exogenous gene would be excreted by the action of the natural bacterial signal peptide out of the microorganism cells. Such utilization of the gene DNA according to the present invention can be appropriately practiced, of course, by referring to the textbooks or literature concerning recombinant DNA technology.

A typical mode of utilization of the DNA gene according to the present invention is that as a plasmid to be used as the vector. Such a plasmid comprises a DNA portion corresponding to the controlling region from alkaline phosphatase and a gene coding for the alkaline phosphatase signal peptide under control of the region, said gene coding for the signal peptide being defined by having a restriction endonuclease recognition site artificially created such that the amino acid sequence coded for by the base sequence is not changed and that the corresponding cleavage site lies at the downstream terminus of said gene.

Thus, the plasmid has the preferred DNA gene of the present invention as described above incorporated into a plasmid having a controlling region of alkaline phosphatase so as to place the gene under control of the region, thereby enabling proliferation within the host microorganism.

A specific example of this plasmid has the codon (x) at the downstream terminus of the natural bacterial signal peptide gene corresponding to the gene coding for the signal peptide of the plasmid pYK [this plasmid is on deposit as E. coli K12c600 containing this plasmid, namely E. coli K12c600 (pYK 283), Fermentation Research Institute, Agency of Industrial Science & Technology, Japan (FERM-BP 556)] and the initial codon (y) of the DNA portion linked thereto downstream from the codon (x), which codons (x and y) are as shown in FIG. 1. The bacteriological properties of E. coli K12c600 (pYK 283) are the same as those of the host microorganism E. coli K12c600 except for those originating from the plasmid pYK 283 contained therein, and some of its specific properties are described in the document annexed to the sample of the microorganism deposited at Fermentation Research Institute, Agency of Industrial Science & Technology.

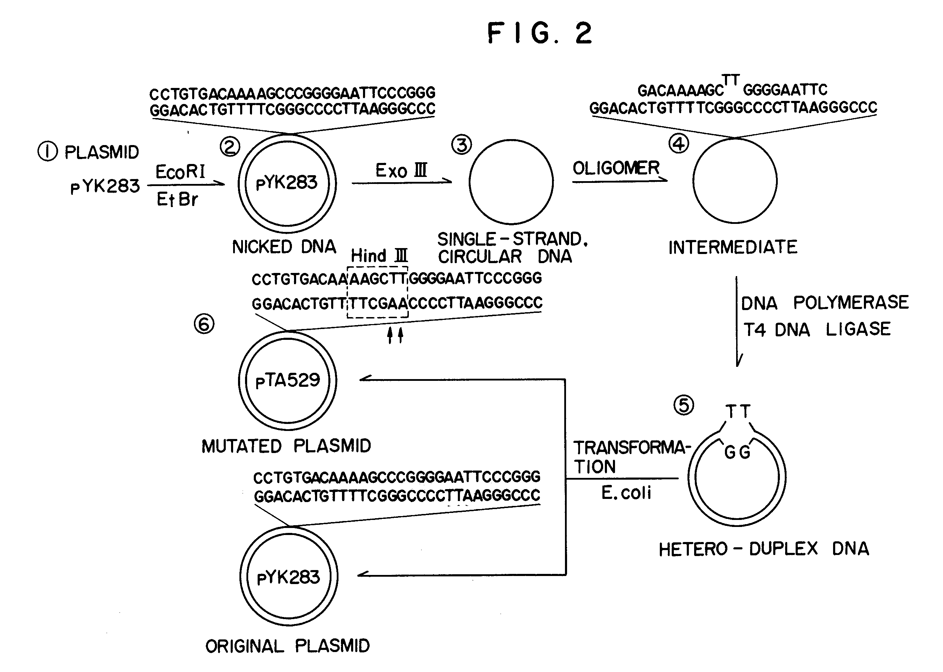

The plasmid PTA 529 can be produced according to the process as shown in FIG. 2. It should be understood, however, that there are other processes than that shown in FIG. 2, and the plasmids incorporating other DNA genes of the present invention can also be produced similarly.

Now, referring to FIG. 2, the plasmid pYK 283 ( ① ) is subjected to the restriction endonuclease EcoRI

treatment in the presence of ethidium bromide (EtBr) to form a nicked DNA ②, which is a double-stranded cyclic DNA having a nick on one strand, and the nicked plasmid ② is then subjected to the exonuclease III digestion to form a single stranded DNA ( ③ ). The single-stranded DNA thus obtained is then processed into a hetero-duplex DNA ⑤ through the action thereto of DNA polymerase I with a use as a primer ④ of a synthetic oligonucleotide for inducing point mutation previously synthesized into a double stranded DNA, which is then converted into the hetero-duplex DNA ⑤ upon the action of DNA ligase (T4 DNA ligase). Then, the hetero-duplex is introduced into E. coli K 12c600, following the method of Kuschner (Genetic Engineering, 1978, 17 (1978)) to transform the host, and strains which have become ampicillin resistant are obtained ( ⑥ ).

From among these strains, the desired mutant strain is obtained upon colony hybridization (Methods in Enzymology, 68, 379, Academic Press INc., New York, 1979). Finally, the plasmid is taken out of this strain, and it is confirmed according to the Maxam-Gilbert method (Methods in Enzymology, Vol. 65, Nucleic Acid Part I, 499 - 560 (1980), Academic Press) whether or not the desired sequence is formed to obtain the plasmid pTA 529.

As to the details concerning the preparation of the plasmid, reference is made to the experimental examples as shown below. As the method for synthesis of the oligonucleotide as the primer referred to hereinabove, reference may be made to the diester method (Science, 203, 614 (1979)), the triester method (Nucleic Acids Research 8, 2331 (1980), ibid 8, 5193 (1980), ibid 8, 5491 (1980)), or the solid phase method (Nature, 281, 18 (1979), Biochemistry 1980, 6096 (1980)), the liquid phase method or the method employing an enzyme (Nucleic Acids Research, 8, 5753 (1980)), etc. In the case of the present invention, the solid phase method is preferred.

Experimentation

(1) Synthesis of oligonucleotide

As an oligonucleotide for inducing point mutation, a fragment having the following base sequence was synthesized:

$$\text{GA} - \text{CA} - \text{AAA} - \text{GC} - \text{TT} - \text{GG} - \text{GG} - \text{AA} - \text{TT} - \text{C} - - \text{\textcircled{Ps}}$$

wherein G represents guanine, A adenine, C cytosine, T thymine and --⑭s polystyrene.

With the use of 30 mg of a 1% polystyrene resin to which cytosine had been bonded, a 3'-terminal diester type nucleotide (20 mg of a monomer, 25 mg of a dimer and 35 mg of a trimer), and 20 mg of a condensing agent mesitylene sulfonyl triazolide (hereinafter referred to as MSNT), condensation was successively repeated to obtain a desired chain length according to the method of Nucleic Acid Research,8, 5491 (1980). The overall condensation yield was found to be 35%. After completion of the final condensation step, the resin was dried and 300 $\mu$l of a solution of 0.5 M picolinealdoxime-tetramethylguanidine in pyridine-water (9:1) was added thereto, and the mixture was left to stand overnight at 37°C. Then, conc. ammonia water (4 ml) was added, and after the mixture was left to stand overnight at 55°C, the resin was removed by filtration. The filtrate was dissolved in water and subjected to extraction with ether (deprotection). Subsequently, the ether extract was subjected to gel filtration with Sephadex® G-50 (eluent: 50 mM triethylammonium bicarbonate, pH 7.5). During the gel filtration, the respective fractions were subjected to measurement of absorption at 260 nm, and the peak eluted first was collected and subjected to high performance liquid chromatography (HPLC), followed by reaction in 2 ml of 80% acetic acid at room temperature for 10 minutes for conversion into a 5'-hydroxyl derivative to obtain the desired synthetic segment. As the column, $\mu$ Bondapak®C 18 (reverse phase) was employed. The base sequence of the oligonucleotide synthesized was confirmed according to the Maxam-Gilbert method.

As the next step, 300 pmol of the fragment synthesized was freeze-dried and allowed to react with 1.5 $\mu$l of polynucleotide kinase (15 units) in 20 $\mu$l of a solution of 50 mM Tris-hydrochloride buffer (pH 7.5), 10 mM magnesium chloride, 5 mM DTT (dithiothreitol) and 1.25 mM ATP at 37°C for 2 hours (phosphorylation), which step was followed by boiling at 90°C for 2 minutes to terminate the phosphorylation reaction. Then, the reaction mixture was desalted with Sephadex® G-50 to obtain the desired oligomer.

(2) Induction of point mutation

The pYK 283 (plasmid) (10 $\mu$g) was dissolved in 100 $\mu$l of a solution of 100 mM Tris-hydrochloride buffer (pH 7.5), 7 mM magnesium chloride, 50 mM sodium chloride, 7 mM mercaptoethanol, 120 $\mu$g/ml ethidium bromide and 0.01% Nonidet (registered trade mark) P-40, and 2 $\mu$l of EcoRI (10,000 U/1.67 ml) was added thereto to carry out reaction at 37°C for one hour. After removal of the proteins by addition of phenol-chloroform (1:1), ethanol precipitation was conducted. The precipitate was dissolved in 50 $\mu$l of a solution of 10 mM Tris-hydrochloride buffer (pH 7.5), 7 mM magnesium chloride, 100 mM sodium chloride, 7 mM mercaptoethanol and allowed to react with 1.5 $\mu$l of Exonuclease III (5000 U/107 $\mu$l) at 37°C for one hour.

After completion of the reaction, 1.5 $\mu$l of Hinf I (5000 U/883 $\mu$l) and 1 $\mu$l of bacterial alkaline phosphatase (BAP) (10,000 U/39 $\mu$l) were added to the solution to carry out reaction for one hour, and, after removal of the proteins by addition of phenol-chloroform (1:1), ethanol precipitation was conducted. To the precipitate were added 3 $\mu$l of E. coli DNA polymerase I larger fragment (200 U/134 $\mu$l) and 3 $\mu$l of T4 DNA ligase (200 U/80 $\mu$l) in a solution of 60 pmol of the oligonucleotide phosphorylated at the 5'-terminus with 2 $\mu$l of polynucleotide kinase (200 U/20 $\mu$l) as synthesized above, 20 mM Tris-hydrochloride buffer (pH 7.5), 10 mM magnesium chloride, 10 mM DTT, 750 $\mu$M each of dATP, dGTP, dCTP and TTP, and 1 mM ATP, and the reaction was carried out overnight at 14°C. After removal of the proteins with addition of phenol-chloroform (1:1), ethanol precipitation was conducted to obtain a closed circular DNA.

### (3) Transformation

By use of the above DNA in an amount equivalent to about 2 $\mu$g as the DNA amount of the pYK 283, transformation of E. coli K12c600 was performed according to the method of Kuschner. More specifically, after the E. coli K12c600-strain was cultured in 2 ml of L-medium to 0.3 - 0.4 OD/550 nm, the microbes were collected and suspended under ice-cooling in 1 ml of a solution of 10 mM MOPS (3-[N-morpholino]-propanesulfonic acid) (pH 7.0), 10 mM rubidium chloride, which step was followed immediately by collection of the microbes. Subsequently, the microbes were suspended in 1 ml of a solution of 100 mM MOPS (pH 6.5), 10 mM rubidium chloride, 50 mM calcium chloride and left to stand in an ice-bath for 30 minutes.

Again, after the microbes had been collected, they were suspended in 0.2 ml of a solution of 100 mM MOPS (pH 6.5), 10 mM rubidium chloride, 50 mM calcium chloride, and 3 $\mu$l of DMSO (dimethyl sulfoxide) and DNA (the reaction mixture after induction of point mutation, recovered by ethanol precipitation) were added thereto, and the mixture was further left to stand in an ice-bath for 30 minutes. Then, after heating at 45°C for one minute, 2 ml of L-medium was added, and the mixture was left to stand at room temperature for one hour. Thereafter 1200 strains which became ampicillin resistant were obtained on an L-plate (1% trypton, 0.5% yeast extract, 0.5% sodium chloride, 1.5% agar) containing ampicillin.

### (4) Cloning of mutant strain

Cloning was conducted by colony hybridization. More specifically, 550 strains of the above 1200 ampicillin resistant strains were transferred to a nitrocellulose filter, cultured overnight on L-plate, then transferred to an L-plate containing Spectinomycin (300 $\mu$g/ml), and further cultured overnight. The filter was immersed in 0.5N sodium hydroxide for 15 minutes and washed twice with 0.5 M Tris-hydrochloride buffer (pH 7.5), 1.5 M sodium chloride. Then, after washing with 0.03 M sodium-citrate, 0.3 M sodium chloride, the filter was dried at 60°C for one hour, and further dried in a vacuum oven at 80°C for 2 hours. The filter was immersed overnight at 37°C in a solution (6 ml) containing 0.9 M sodium chloride, 0.09 M Tris-hydrochloride buffer (pH 7.5), 0.006 M EDTA, 0.1 % Ficoll, 0.1% polyvinyl pyrrolidone (PVP), 0.1% bovine serum albumine (BSA), 0.5% SDS, 10% dextran sulfate and 100 $\mu$g/ml of E. coli DNA.

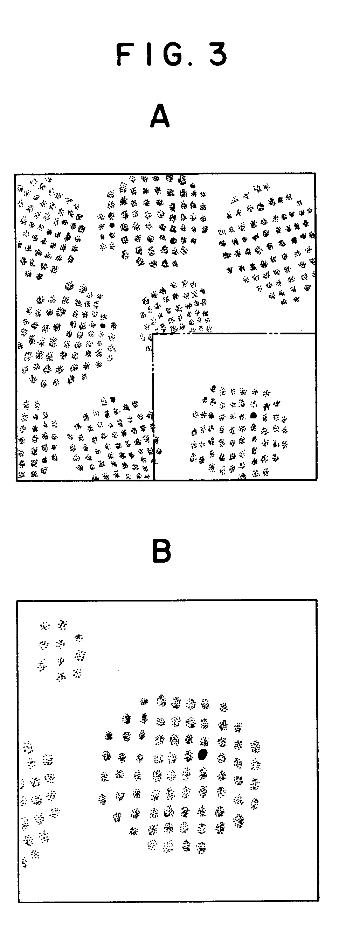

To the filter was added 2,500,000 cpm/ml of the product obtained by labelling 15 pmol of the previously synthesized oligonucleotide with ($\gamma$-32P)ATP, and the filter was further left to stand at 37°C for 2 days. Then, the filter was washed with 0.9 M sodium-citrate, 0.9 M sodium chloride at 42°C three times each for 3 minutes, after which it was dried. Autoradiography of the dried product gave three strains which exhibited positive reactivity. The results of the autoradiography are shown in FIGS. 3A and 3B. A shows the whole result, and B shows a partially enlarged view thereof. The densely black dot in the figures is the mutant strain.

### (5) Confirmation

The plasmid was taken out of the three strains obtained and hydrolyzed with the restriction endonuclease Hind III, whereby it was confirmed whether or not the restriction endonuclease recognition site was created. The plasmid was prepared by culturing the transformed strain in the presence of 25 $\mu$g/ml of

ampicillin in 5 ml of L-medium to 0.4 OD/550 nm, and Spectinomycin was added to a final concentration of 300 μg/ml, after which the culture was left standing overnight. Of the cultured microorganism broth, 2 ml was collected and suspended by addition of 100 μl of a solution containing 2 mg/ml lysozyme, 50 mM glucose, 10 mM CDTA (1,2-diaminocyclohexane-N,N,N',N'-tetraacetic acid), and 25 mM Tris-hydrochloride buffer (pH 8.0), and the suspension was left to stand under ice-cooling for 30 minutes. Then, after centrifugation (5 minutes at 12,000 g), the supernatant was extracted with chloroform-phenol (1:1). Thereafter, 1 ml of ethanol was added to the aqueous layer, and the mixture was left to stand at -70°C for 5 minutes and further subjected to centrifugation (5 minutes at 12,000 g).

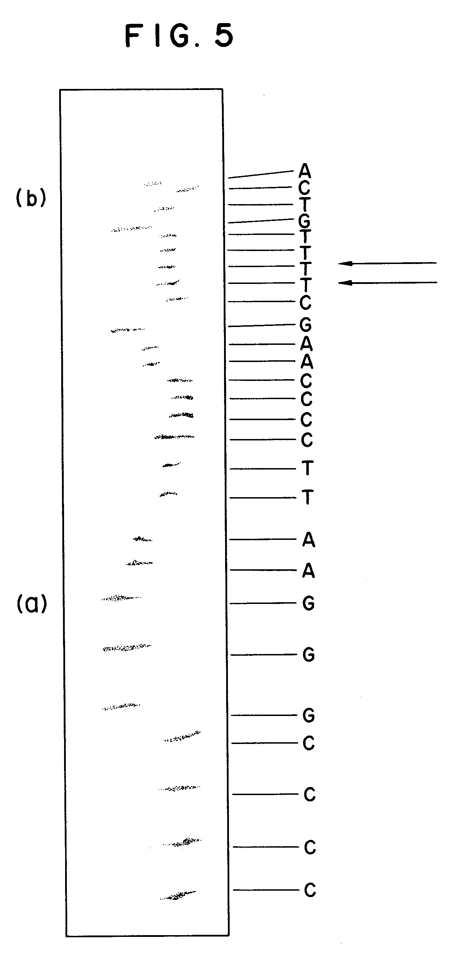

Confirmation of creation of the restriction endonuclease recognition site was performed by 1% Agarose gel electrophoresis, after ethanol precipitation was further repeated twice for the precipitate obtained, and the precipitate was hydrolyzed with Hind III. The result of the electrophoresis is shown in FIG. 4, in which A and B from the left side are those of the plasmid pYK 283, A being obtained by the reaction with Hind III and B being Control. C and D are those of the plasmid of the present invention, C being obtained by the reaction with EcoRI, D with Hind III, and E being Control.

Generally speaking, as the behavior of DNA on gel electrophoresis, treatment of ccc DNA (covalent closed circular DNA) with a restriction enzyme will make it a straight chain DNA, whereby its mobility becomes smaller. In FIG. 4, from A and B, it can be seen that there is no change in the band even by treatment of pYK 283 with the restriction endonuclease Hind III, indicating that there is no cleavage point for Hind III. On the other hand, from C (EcoRI treated), D (Hind III treated), and E (Control), it can be seen that the mobility of those subjected to treatment with restriction endonuclease (C, D) becomes greater, with the band appearing on the ⊖ side than Control (E). Thus, it can be seen that a new restriction endonuclease Hind III recognition site has been created. Also, the base sequence in the vicinity of the Hind III recognition site of this plasmid was confirmed according to the Maxam-Gilbert method (FIG. 5). The base sequence between (a) and (b) in the figure is GAATTCCCCAAGC-TTTTGTCA. In FIG. 5, the arrowheads (←) indicate the modified base sequence. Thus, it can be seen that, as shown by the two plasmids (pTA 529 and pYK 283) in 6 in FIG. 2, only the portion indicated by the arrowhead (↑) is changed, with the result that the restriction endonuclease Hind III recognition site (the portion encircled with the broken line) has been newly created.

## Claims

1. A DNA comprising a gene coding for a natural bacterial signal peptide, said gene differing from the corresponding natural gene by having, towards its downstream end, at least part of a restriction endonuclease recognition site artificially created from the natural gene by using the degeneracy of the genetic code so that the same amino acid sequence is encoded.

2. A DNA according to Claim 1, wherein the cleavage site within the restriction endonuclease recognition site lies at the downstream terminus of said gene.

3. A DNA according to Claim 1, wherein the restriction endonuclease recognition site lies upstream from the downstream terminus of said gene and the portion of the gene between said recognition site and said downstream terminus is such that the natural bacterial signal peptide is still encoded.

4. A DNA according to any one of Claims 1-3 further comprising an exogenous gene directly linked to the downstream terminus of the signal peptide gene.

5. A DNA according to Claim 1, wherein the gene coding for a natural bacterial signal peptide originates from an alkaline phosphatase, the restriction endonuclease recognition site is the Hind III recognition site, and the restriction endonuclease cleavage site is located at the downstream terminus of said gene.

6. A process for producing a DNA as defined in claim 1 comprising the steps of:
   (a) providing a DNA containing a gene coding for a natural bacterial signal peptide; and
   (b) modifying the base sequence of said gene towards its downstream end without changing the amino acid sequence corresponding to said base sequence, thereby creating a restriction endonuclease recognition site at least a part of which is located on said gene.

7. The process of Claim 6 wherein (b) is followed by
   (c) cleaving the DNA with the restriction endonuclease recognized by said site, thereby obtaining a cleaved end of said restriction endonuclease;

(d) providing another DNA fragment having the same restriction endonuclease cleaved end on its upstream terminus, followed, if necessary, by a DNA portion useful to reconstitute the gene for the natural bacterial signal peptide, the latter being followed by an exogenous gene; and

(e) linking the DNA fragments from the above steps (c) and (d) to each other at the restriction endonuclease cleaved ends, thereby obtaining a DNA comprising a gene coding for a natural bacterial signal peptide and the exogenous gene directly linked to the downstream terminus of the signal peptide gene.

8. The process according to Claim 6 or 7, wherein modification of the base sequence is accomplished by point mutation with the use of a synthetic fragment.

9. A plasmid comprising the controlling region derived from an alkaline phosphatase gene and a gene coding for the alkaline phosphatase signal peptide, said gene coding for the signal peptide being defined by having a restriction endonuclease recognition site artificially created such that the amino acid sequence coded for by the base sequence is not changed and that the corresponding cleavage site lies at the downstream terminus of said gene.

10. The plasmid pTA 529 containing the sequence as shown in Fig. 1 which plasmid is obtainable from plasmid pYK 283 (FERM BP-556) as shown in Fig. 2.

**Patentansprüche**

1. DNA, umfassend ein Gen, das für ein natürliches bakterielles Signalpeptid codiert, wobei das Gen sich von dem entsprechenden natürlichen Gen dadurch unterscheidet, daß es gegen sein Stromabwärtsende mindestens einen Teil einer Erkennungsstelle einer Restriktions-Endonuclease, die künstlich aus dem natürlichen Gen unter Verwendung der Degeneriertheit des genetischen Codes geschaffen wurde, so daß die gleiche Aminosäuresequenz codiert wird, besitzt.

2. DNA nach Anspruch 1, dadurch **gekennzeichnet,** daß die Schnittstelle in der Erkennungsstelle für die Restriktions-Endonuclease am Stromabwärtsende dieses Gens liegt.

3. DNA nach Anspruch 1, dadurch **gekennzeichnet,** daß die Erkennungsstelle für die Restriktions-Endonuclease stromaufwärts von dem Stromabwärtsende des Gens liegt und daß der Teil des Gens zwischen der Erkennungsstelle und dem Stromabwärtsende so ist, daß das natürliche bakterielle Signalpeptid noch codiert wird.

4. DNA nach einem der Ansprüche 1 bis 3, dadurch **gekennzeichnet,** daß es weiterhin ein exogenes Gen in direkter Bindung an das Stromabwärtsende des Signalpeptid-Gens enthält.

5. DNA nach Anspruch 1, dadurch **gekennzeichnet,** daß das Gen, das für ein natürliches bakterielles Signalpeptid codiert, aus einer alkalischen Phosphatase stammt, die Erkennungsstelle für die Restriktions-Endonuclease die Hind-III-Erkennungsstelle ist und die Spaltstelle für die Restriktions-Endonuclease am Stromabwärtsende des Gens lokalisiert ist.

6. Verfahren zur Herstellung einer DNA nach Anspruch 1, dadurch **gekennzeichnet,** daß man

(a) eine DNA, die ein Gen, das für ein natürliches bakterielles Signalpeptid codiert, enthält, bereitstellt und

(b) die Basensequenz des Gens gegen sein Stromabwärtsende ohne Veränderung der Aminosäuresequenz entsprechend der Basensequenz modifiziert, wodurch eine Erkennungsstelle für eine Restriktions-Endonuclease geschaffen wird, von der mindestens ein Teil auf dem Gen lokalisiert ist.

7. Verfahren nach Anspruch 6, dadurch **gekennzeichnet,** daß man nach Stufe (b)

(c) die DNA mit der Restriktions-Endonuclease, die von dieser Stelle erkannt wird, schneidet, wodurch ein geschnittenes Ende der Restriktions-Endonuclease erhalten wird,

(d) ein weiteres DNA-Fragment bereitstellt, das ein durch die gleiche Restriktions-Endonuclease geschnittenes Ende an seinem Stromaufwärtsende besitzt, an das sich, sofern notwendig, ein DNA-Teil anschließt, der nützlich ist, um das Gen für das natürliche bakterielle Signalpeptid zu rekonstituieren, wobei auf das letztere ein exogenes Gen folgt, und

(e) die DNA-Fragmente aus den obigen Stufen (c) und (d) aneinander an den durch die Restriktions-Endonuclease geschnittenen Enden bindet, wodurch eine DNA erhalten wird, die ein Gen, das für ein natürliches bakterielles Signalpeptid codiert, und das exogene Gen, das direkt an das Stromabwärtsende des Signalpeptid-Gens gebunden ist, umfaßt.

8. Verfahren nach Anspruch 6 oder 7, dadurch **gekennzeichnet,** daß die Modifikation der Basensequenz durch Punktmutation unter Verwendung eines synthetischen Fragments erzielt wird.

9. Plasmid, umfassend die Kontrollregion, die sich von einem Gen für die alkalische Phosphatase ableitet, und ein Gen, das für das Signalpeptid der alkalischen Phosphatase codiert, wobei das Gen, das für das Signalpeptid codiert, so definiert ist, daß es eine künstlich geschaffene Erkennungsstelle für eine Restriktions-Endonuclease besitzt, so daß die Aminosäuresequenz, die durch die Basensequenz codiert wird, nicht verändert wird, und daß die entsprechende Schnittstelle am Stromabwärtsende des Gens liegt.

10. Plasmid pTA 529, enthaltend die in Fig. 1 gezeigte Sequenz, wobei das Plasmid aus Plasmid pYK 283 (FERM BP-556), wie in Fig. 2 gezeigt, erhältlich ist.

**Revendications**

1. ADN comprenant un gène codant pour un peptide signal bactérien naturel, ledit gène différant du gène naturel correspondant par le fait qu'il présente, en direction de son extrémité aval, au moins une partie d'un site de reconnaissance par une endonucléase de restriction, créée artificiellement à partir du gène naturel par l'utilisation de la dégénérescence du code génétique de telle sorte que la même séquence d'acides aminés soit codée.

2. ADN selon la revendication 1, dans lequel le site de clivage au sein du site de reconnaissance par une endonucléase de restriction se situe à l'extrémité terminale aval dudit gène.

3. ADN selon la revendication 1, dans lequel le site de reconnaissance par une endonucléase de restriction se situe en amont de l'extrémité terminale aval dudit gène, et la portion du gène entre ledit site de reconnaissance et ladite extrémité terminale aval est telle que le peptide signal bactérien naturel soit toujours codé.

4. ADN selon l'une quelconque des revendications 1 à 3, comprenant en outre un gène exogène directement lié à l'extrémité terminale aval du gène du peptide signal.

5. ADN selon la revendication 1, dans lequel le gène codant pour un peptide signal bactérien naturel provient d'une phosphatase alcaline, le site de reconnaissance par une endonucléase de restriction est le site de reconnaissance par HindIII, et le site de clivage par une endonucléase de restriction est situé à l'extrémité terminale aval dudit gène.

6. Procédé de fabrication d'un ADN tel que défini à la revendication 1, comprenant les étapes consistant à :
(a) prendre un ADN contenant un gène codant pour un peptide signal bactérien naturel ; et
(b) modifier la séquence de bases dudit gène en direction de son extrémité aval sans modifier la séquence d'acides aminés correspondant à ladite séquence de bases, créant ainsi un site de reconnaissance par une endonucléase de restriction dont au moins une partie est située sur ledit gène.

7. Procédé selon la revendication 6, dans lequel l'étape (b) est suivie par les étapes consistant à :
(c) cliver l'ADN avec l'endonucléase de restriction reconnue par ledit site, pour obtenir ainsi une extrémité clivée de ladite endonucléase de restriction ;
(d) prendre un autre fragment d'ADN ayant la même extrémité clivée par l'endonucléase de restriction sur son extrémité terminale amont, suivi, si nécessaire, par une portion d'ADN utile pour reconstituer le gène pour le peptide signal bactérien naturel, cette dernière étant suivie d'un gène exogène ; et

(e) lier l'un à l'autre les fragments d'ADN des étapes (c) et (d) ci-dessus au niveau des extrémités clivées par l'endonucléase de restriction, pour obtenir ainsi un ADN comprenant un gène codant pour un peptide signal bactérien naturel et le gène exogène directement relié à l'extrémité terminale aval du gène du peptide signal.

8. Procédé selon la revendication 6 ou 7, dans lequel la modification de la séquence de bases est accomplie par une mutation ponctuelle avec l'utilisation d'un fragment synthétique.

9. Plasmide comprenant la région de contrôle issue d'un gène de la phosphatase alcaline et d'un gène codant pour le peptide signal de la phosphatase alcaline, ledit gène codant pour le peptide signal étant défini comme ayant un site de reconnaissance par une endonucléase de restriction créé de façon artificielle de telle sorte que la séquence d'acides aminés qui est codée par la séquence de bases ne soit pas modifiée et que le site de clivage correspondant se situe à l'extrémité terminale aval dudit gène.

10. Plasmide pTA 529 contenant la séquence telle que représentée sur la Figure 1, lequel plasmide peut être obtenu à partir du plasmide pYK 283 (FERM BP-556) tel que représenté sur la Figure 2.

# F I G. I

# F I G. 4

# FIG. 2

CCTGTGACAAAAGCCCGGGGAATTCCCGGG
GGACACTGTTTTCGGGCCCCTTAAGGGCCC

① PLASMID

pYK283 —EcoRI/EtBr→ ② pYK283

NICKED DNA

—Exo Ⅲ→ ③ SINGLE-STRAND. CIRCULAR DNA

—OLIGOMER→ ④ INTERMEDIATE

GACAAAAGC^TT GGGGAATTC
GGACACTGTTTT CGGGCCCCTTAAGGGCCC

DNA POLYMERASE
T4 DNA LIGASE

⑤ HETERO-DUPLEX DNA
TT
GG

TRANSFORMA-TION
E.coli

Hind Ⅲ
CCTGTGACAA AAGCTT GGGGAATTCCCGGG
GGACACTGTT TTCGAA CCCCTTAAGGGCCC

⑥ pTA529

MUTATED PLASMID

CCTGTGACAAAAGCCCGGGGAATTCCCGGG
GGACACTGTTTTCGGGCCCCTTAAGGGCCC

pYK283

ORIGINAL PLASMID

EP 0 133 321 B1

# FIG. 3

## A

## B

# F I G. 5